Europäisches Patentamt

European Patent Office

Office européen des brevets

(11) Numéro de publication: **0 283 387**
**A1**

## (12) DEMANDE DE BREVET EUROPEEN

(21) Numéro de dépôt: 88400560.4

(22) Date de dépôt: 10.03.88

(51) Int. Cl.⁴: **A 61 B 5/10**
A 61 B 9/00, A 61 N 1/36

(30) Priorité: **13.03.87 FR 8703785**
**13.03.87 FR 8703786**

(43) Date de publication de la demande:
**21.09.88 Bulletin 88/38**

(84) Etats contractants désignés:
**AT BE CH DE ES FR GB GR IT LI LU NL SE**

(71) Demandeur: **BIO INDUSTRY SARL**
**Z.I. de la Liane "Résurgat 1"**
**F-62230 Outreau (FR)**

(72) Inventeur: **Bournonville, Patrick**
**Les Calhandes**
**F-62250 Marquise (FR)**

(74) Mandataire: **Ecrepont, Robert**
**Cabinet Ecrepont 12 Place Simon Vollant (Porte de Paris)**
**F-59800 Lille (FR)**

(54) Appareil de contrôle de la réaction neuro-musculaire d'un patient.

(57) L'invention se rapporte à un appareil de contrôle de la réaction neuro-musculaire d'un patient notamment mais non exclusivement dans le but d'assurer un monitorage de la curarisation et de la décurarisation.

Cet appareil est caractérisé en ce que le piston du capteur (3) est associé au corps au moins sensiblement cylindrique de manière à coulisser sensiblement radialement à l'axe du corps pour, quelles que soient les dimensions de la main du patient, permettre l'actionnement du piston (35) du capteur non plus par la dernière mais la première phalange (10) du pouce (4) de la main (5) équipée du dispositif (1) et en ce que :
- le dit corps est associé à au moins une butée (7) disposée pour, en coopérant avec au moins l'un des organes que sont la main et l'avant bras du patient, établir une référence pour la position de la main (5) au long de l'axe longitudinal (8) de la poignée (6), et
- le dispositif comprend au moins un moyen (9) permettant d'ajuster sensiblement au long de l'axe (8) de la poignée (6), la position relative des éléments que sont la butée (7) et l'organe de détection (3).

Application à l'industrie du matériel médical.

Fig. 2

**Description**

## APPAREIL DE CONTROLE DE LA REACTION NEURO-MUSCULAIRE D'UN PATIENT

L'invention se rapporte à un appareil de contrôle de la réaction neuro-musculaire d'un patient notamment mais non exclusivement dans le but d'assurer un monitorage de la curarisation et de la décurarisation.

Il est maintenant clairement établi que l'importance et la durée de l'effet d'un curare donné sont assez largement variables d'un malade à l'autre.

Si l'on veut donc injecter au malade la quantité de curare nécessaire à la réalisation de l'acte chirurgical et qui soit en même temps la plus faible possible, il est nécessaire de pouvoir mesurer le degré de curarisation.

Par ailleurs, la sécurité du réveil repose sur la plus juste appréciation possible de la décurarisation (donc de la curarisation résiduelle) qu'il est tout aussi souhaitable de chercher à quantifier chaque fois qu'on le peut.

La quantification de la curarisation peut être obtenue par contrôle de la réponse musculaire secondaire à la stimulation du nerf moteur.

A ce jour, on connaît des appareils réalisant ce contrôle (FR-A-1359777).

Un tel appareil comprend principalement :
- un stimulateur électrique comprennant lui-même un générateur d'impulsions relié à des électrodes appliquées au patient au niveau d'un des points de passage du nerf moteur choisi,
- un capteur de la réponse musculaire à surveiller avec les moyens en vue de son adaptation au patient,
- un analyseur du signal émis par le capteur.

Le simulateur et l'analyseur sont évidemment de préférence conçus de manière à pouvoir respectivement émettre des stimulations différentes et analyser les réponses à ces différentes stimulations.

Pour la stimulation électrique, on agit généralement au niveau d'un de ses nerfs cubitaux et la transmission de l'influx électrique est assurée par des organes à cet effet tels des électrodes ou des aiguilles sous cutanées qui, reliées à l'appareillage, sont disposées par exemple au niveau d'un des poignets du dit patient.

Pour la détection et la mesure de la réponse musculaire, dans les réalisations connues à ce jour, on dispose le capteur de manière à détecter et mesurer la force d'adduction au niveau de la dernière phalange du pouce du membre au niveau duquel est organisée la stimulation.

Pour organiser correctement la détection au niveau de la main concernée du patient, il est nécessaire d'immobiliser cette dernière et de prépositionner le capteur.

A cet effet, on connait un dispositif (US-A-4.326.528) qui permet d'immobiliser l'un des avant-bras du patient en position main à plat.

Outre une plaque support contre laquelle est placée la main, le dispositif comprend des moyens de fixation de l'avant bras et de la main sur la plaque, tels des sangles, et l'organe de détection associé à la plaque par un moyen de réglage de sa position permettant donc d'adapter le dispositif aux dimensions de la main du patient.

Outre son aspect traumatisant pour le patient et son encombrement important, on lui reproche de contraindre le muscle adducteur de la dernière phalange du pouce à travailler dans un plan notamment défini par la plaque support et en tous cas notablement éloigné du plan d'adduction naturelle de cet organe ce qui en altère les réactions d'adduction.

Dans un autre dispositif, le capteur consiste en un piston coulissant axialement dans un corps cylindrique enserré par la main maintenue fermée par des sangles et agissant sur une jauge de contrainte.

S'il ressort le problème de la mauvaise position, cette réalisation impose également d'agir sur le piston par la dernière phalange du pouce, aussi on reproche à ces deux dispositifs un manque de fiabilité dû aux nombreux mouvements parasites de la dernière phalange du pouce et aux parasites dans l'environnement du patient tels par exemple l'action d'un bistouri électrique ou des chocs mécaniques.

Les signaux captés et analysés dans les appareils connus à ce jour sont alors, pour une part importante, constitués par des signaux parasites parmi lesquels il est parfois difficile de reconnaître ceux correspondant aux réponses musculaires attendues, lesquelles peuvent même être noyées dans la vague des signaux parasites qui, comme les autres, se présentent sous la forme de pics plus ou moins prononcés d'où un risque d'erreurs extrêmement important dans les résultats de l'analyse.

Pour en limiter les effets, on connait (MEDICAL & BIOLOGICAL ENGINEERING & COMPUTING volume 23 N° 6 de novembre 1985 pages 547-555 IFMBE Londres, GB H.S BRADLOW et al "microcomputer-based muscle relaxation monitor and controller for clinical use") un dispositif dont le circuit d'analyse et le stimulateur sont reliés afin de ne lire le signal capté qu'au moment où il est attendu.

Si entretemps, on évite de mal interpréter de simples parasites, pendant la lecture, les parasites subsistent et faussent cette lecture.

On connaît également (US-A-4.387.723) un dispositif dans lequel on élimine systématiquement une bande de fréquence d'environ 10 Hz des signaux reçus ce qui élimine les parasistes correspondants mais ne tient pas compte de l'évolution de bruits et soit des parasites subsistent, soit les signaux captés sont amputés sans raison.

Par ailleurs, ne serait-ce que pour une plus grande linéarité de la réponse captée, au lieu d'être simplement appliqué contre l'organe où la réponse est attendue, le piston du capteur est généralement appuyé sur le dit organe avec une certaine force dite "pré-charge" qui est fonction de différents facteurs tels la sensibilité du patient aux impulsions.

Malheureusement, ces facteurs peuvent euxmêmes évoluer pendant le temps que dure l'analyse et fausser le résultat.

Un résultat que l'invention vise à obtenir est un appareil moins traumatisant et qui fasse travailler l'organe dans son plan normal d'adduction .

Est aussi un résultat de l'invention, un tel appareil moins soumis à des mouvements parasites de l'organe.

Un autre résultat que l'invention vise à obtenir est un appareil du type précité protégé de l'influence des bruits de l'environnement.

Est également un résultat que l'invention vise à obtenir est un tel appareil qui permette une adaptation en fonction de la modification prévisible de la pré-charge nécessaire.

A cet effet, l'invention a pour objet un appareil du type cité plus haut, comprenant au moins un dispositif permettant de maintenir en position adéquate le capteur permettant de détecter le mouvement d'adduction du pouce d'une des mains, lequel organe comprend un piston coulissant par rapport à un corps au moins sensiblement cylindrique et enserré par la main du patient à la manière d'une poignée, cet appareil étant caractérisé en ce que le piston du capteur est associé au corps au moins sensiblement cylindrique de manière à coulisser sensiblement radialement à l'axe du corps pour, quelles que soient les dimensions de la main du patient, permettre l'actionnement du piston du capteur non plus par la dernière mais la première phalange du pouce de la main équipée du dispositif et en ce que :
- le dit corps est associé à au moins une butée disposée pour, en coopérant avec au moins l'un des organes que sont la main et l'avant bras du patient, établir une référence pour la position de la main au long de l'axe longitudinal de la poignée, et
- le dispositif comprend au moins un moyen permettant d'ajuster sensiblement au long de l'axe de la poignée, la position relative des éléments que sont la butée et l'organe de détection.

L'invention sera mieux comprise à l'aide de la description ci-après faite à titre d'exemple non limitatif en regard du dessin ci-annexé qui représente :

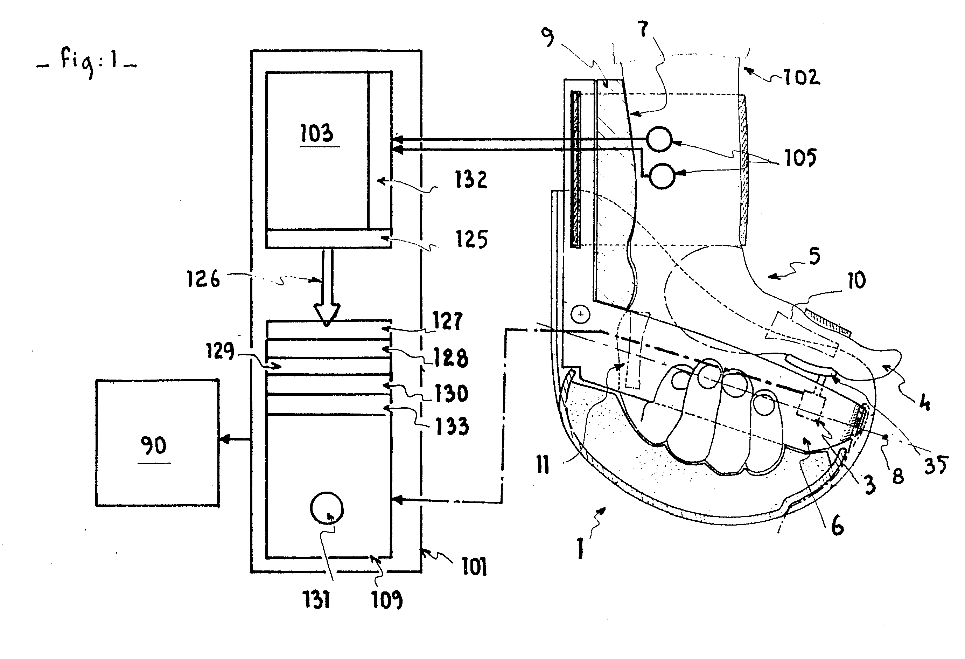

- figure 1 : le schéma bloc de l'appareil,

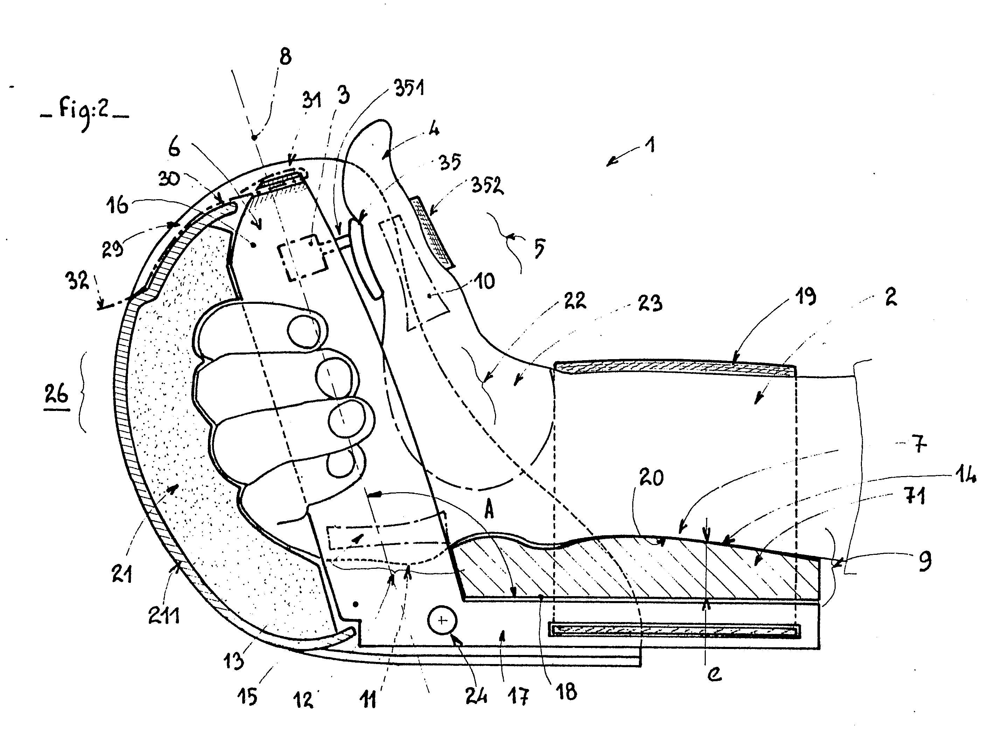

- figure 2 : le dispositif vu en coupe montrant la position de la main et des doigts sur la poignée,

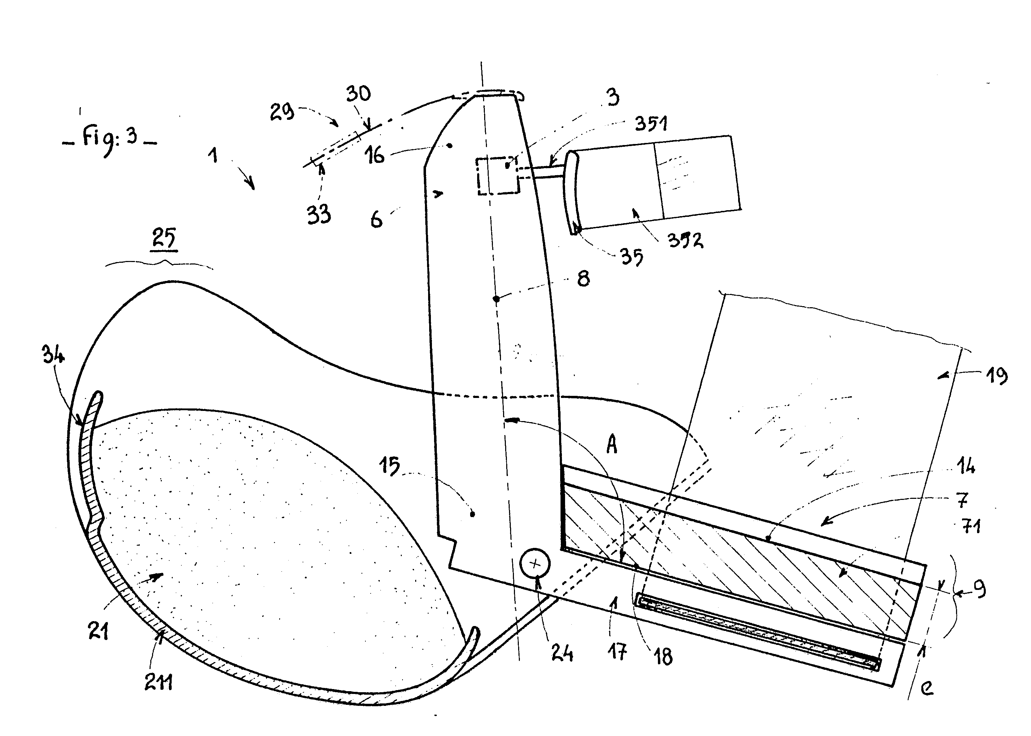

- figure 3 : le dispositif vu selon le même plan de coupe en position dégagée.

En se reportant au dessin, on voit un appareil 101 de contrôle de la réaction neuro-musculaire d'un patient 102 lequel appareil est par exemple utilisable dans le but d'assurer un monitorage de la curarisation et/ou de la décurarisation au moyen :
- d'un stimulateur 103 comprenant lui-même un générateur d'impulsions et, reliées au générateur, des électrodes 105 appliquées au patient 102 au niveau d'un des points de passage du nerf moteur choisi tel le nerf cubital,
- d'un capteur 3 de la réponse musculaire à surveiller comprenant une jauge de contrainte avec ses moyens d'adaptation à l'organe 4 sur lequel on veut capter la réponse,
- d'un analyseur 109 du signal émis par le capteur 3, lesquels stimulateur 103 et analyseur 109 comprennent des moyens leur permettant d'émettre des stimulations différentes et d'analyser les réponses à ces stimulations différentes.

En toute hypothèse, l'appareil comprend au moins un dispositif 1 permettant de maintenir en position adéquate le capteur 3 permettant de détecter le mouvement d'adduction du pouce 4 d'une des mains 5, lequel capteur comprend un piston 35 coulissant par rapport à un corps 6 au moins sensiblement cylindrique et enserré par la main du patient à la manière d'une poignée.

Selon une caractéristique essentielle de l'invention, cet appareil est caractérisé en ce que le piston 35 du capteur 3 est associé au corps 6 au moins sensiblement cylindrique de manière à coulisser sensiblement radialement à l'axe 8 du corps 6 pour, quelles que soient les dimensions de la main du patient, permettre l'actionnement du piston 35 du capteur non plus par la dernière mais par la première phalange 10 du pouce 4 de la main 5 équipée du dispositif 1 et en ce que :
- le dit corps 6 est associé à au moins une butée 7 disposée pour, en coopérant avec au moins l'un des organes que sont la main et l'avant bras du patient, établir une référence pour la position de la main 5 au long de l'axe longitudinal 8 de la poignée 6, et
- le dispositif comprend au moins un moyen 9 permettant d'ajuster, sensiblement au long de l'axe 8 de la poignée 6, la position relative des éléments que sont la butée 7 et le capteur 3.

Suivant l'invention, la butée 7 coopère au moins localement avec la tranche 11 de la main équipée du dispositif notamment dans la zone 12 du métacarpien de l'auriculaire 13 et ce afin d'établir la position de cette main comme précédemment définie.

Dans un mode préféré de réalisation, la butée 7 consiste en un talon 71 longiligne dont la surface d'appui 14 contre le membre du patient s'étend au delà de la poignée 6 et coopère tant avec la tranche 11 de la main qu'avec une fraction de l'avant bras située dans son prolongement.

Dans un autre mode de réalisation, c'est le talon longiligne 71 qui constitue le moyen d'ajustement de la position relative du piston 35 et de la butée 7 sensiblement selon l'axe longitudinal 8 de la poignée et, à cet effet, il est choisi d'une épaisseur "e" au moins suffisante pour, en coopérant par sa face 14 avec le flanc de l'avant bras du patient permettre l'actionnement du capteur 3 par la première phalange du pouce du du patient.

Avantageusement, l'axe longitudinal de la poignée 6 forme avec la butée 7 un angle A sensiblement supérieur à quatre vingt dix degrés et, de préférence, sensiblement égal à cent cinq degrés. Pour associer la butée 7 à l'extrémité 15 de la poignée 6 opposée à celle 16 prés de laquelle est agencé le capteur 3, la dite poignée 6 est solidaire d'une platine 17 qui s'étend sensiblement parallèlement à l'axe de l'avant bras.

Cette platine 17, qui reçoit la butée 7 par sa face opposée 18 à celle d'appui 14, porte des moyens 19 pour sa fixation à la fraction de l'avant bras situé en vis à vis de la butée 7.

Suivant l'invention, pour l'adaptation des doigts de la main autour de la poignée, le dispositif comprend un coussin 21 de forme susceptible d'une part d'envelopper largement la main du patient en la contraignant élastiquement et ce au moins au niveau des doigts refermés sur la poignée mais d'autre part de dégager constamment la zone 22 du muscle adducteur 23 du pouce, lequel coussin est combiné à un élément support 211 relié à la poignée par un moyen de liaison 24 assurant le déplacement du coussin 21 au moins entre deux positions extrêmes 25, 26 dont une position 25 dans laquelle le coussin dégage totalement l'accés à la poignée pour sa préhension par le patient et une autre position 26 dans laquelle il enveloppe largement la main du dit patient comme précédemment défini pour maintenir ses doigts élastiquement refermés sur la poignée.

De préférence, plutôt qu'un guidage en translation qui reste néanmoins possible, le moyen de liaison 24 consiste en une articulation 24 dont l'axe est sensiblement orthogonal au plan passant par l'axe longitudinal 8 de la poignée 6.

Avantageusement, le coussin 21 est en matériau synthétique souple alvéolaire.

L'élément support 211 du coussin 21 consiste en un bouclier rigide qui enveloppe extérieurement le dit coussin 21 de manière à pouvoir induire un contact élastique optimal entre ce dernier et les doigts du patient refermés sur la poignée 6.

Les deux positions extrêmes 25, 26 du bouclier 211 sont délimitées par des organes d'arrêt positionnés de manière appropriée sur les deux éléments mobiles que sont le bouclier 211 et l'ensemble que constituent la poignée 6 et la platine longiligne 17 qui lui est associée.

Pour maintenir le bouclier 211 en position 26 enveloppante pour la main du patient équipé du dispositif, ce dernier comprend bien entendu un moyen de verrouillage 29 qui consiste par exemple en un lien 30 dont une extrémité 31 est ancrée au sommet de la poignée 6 et dont l'autre extrémité libre 32 est équipée d'un élément 33 de liaison amovible avec un point donné 34 du bouclier.

Pour constituer l'élément de liaison amovible 33 et son point d'ancrage 34, on utilise des parcelles de tissus auto-grippantes bien connues sous la dénomination VELCRO.

Le piston 35 d'actionnement de la jauge de contrainte comprend un moyen 352 de liaison du pouce au piston.

Avantageusement, ce moyen de liaison consiste en une sangle qui permet d'enserrer le pouce du patient et d'appliquer sur le piston une précharge de préférence réglable.

Avantageusement, le dispositif porte directement les électrodes 105 de stimulation voire même les autres éléments du dispositif que sont tout ou partie du stimulateur et/ou de l'analyseur pris isolément ou avec au moins certains de leurs accessoires tels des moyens de visualisation des signaux captés et/ou un afficheur des résultats de l'analyse.

L'analyseur peut être relié à un appareil d'injection automatique de curare.

Outre, des moyens de visualisation du signal capté et/ou un afficheur des résultats de l'analyse, cet analyseur peut être relié à un ensemble 90 d'appareils périphériques et notamment :

- une interface de communication avec, par exemple, un clavier, une table traçante ,
- un circuit de liaison à un bistouri électrique.

Le stimumateur 103 peut quant à lui comprendre, lorsque ce type d'alimentation est choisi, un moyen de mesure du niveau de charge de la batterie d'alimentation (non représentée) voire une alarme placée sous son contrôle.

L'appareil est par ailleurs pourvu :
- de moyens complémentaires 125, 126 et 127 d'information de l'analyseur 109 par le stimulateur 103 des instants où chacune des stimulations est émise,
- d'un moyen 128 qui, en tenant compte de cette information et du retard entre l'émission d'une stimulation et la réception du signal de réaction correspondant, distingue dans le signal capté, avec une certaine tolérance, les périodes où se situent les éventuels signaux de réaction des autres périodes pendant lesquelles seuls sont captés des signaux parasites.

Selon l'invention, l'appareil comprend en outre :
- un moyen 129 qui, pendant les périodes où seuls des signaux parasites peuvent être captés, mesure le niveau du signal capté et,
- un moyen 130 qui n'élimine du signal capté que la bande correspondant aux signaux de bruits mesurés, lequel moyen est de préférence situé entre le capteur 3 et le moyen 128 de distinction des périodes pour former une boucle de réglage continu.

Cette synchronisation du stimulateur et de l'analyseur et cette filtration permet d'assurer que le signal envoyé aux moyens d'analyse des réponses aux stimulations soit exempt du bruit environnant quelle qu'en soit l'évolution.

Le signal et le résultat des appareils sont donc plus fiables.

Le stimulateur comprend un moyen 131 de réglage de l'intensité du signal émis et ce progressivement pour rechercher le niveau souhaité.

Avantageusement, le stimulateur 103 comprend un moyen de maintien du signal émis non pas à une tension constante mais à une intensité constante et, de ce fait, les réponses demeurent insensibles aux variations d'impédance entre les électrodes 105 et la pâte conductrice ou le patient lui-même.

L'analyseur comprend également un moyen de calibration 133 du signal capté permettant notamment d'adapter à la largeur de l'échelle de lecture un signal de référence propre à chaque cas. Selon une caractéristique de l'invention, il s'agira d'un amplificateur à gain variable à réglage automatique.

Il est évident que l'appareil peut faire l'objet de nombreuses modifications sans pour cela sortir du cadre de la présente invention.

**Revendications**

1. Appareil (101) de contrôle de la réaction neuro-musculaire d'un patient (102) par exemple utilisable dans le but d'assurer un monitorage de la curarisation et/ou de la décurarisation et ce au moyen :

- d'un stimulateur (103) comprenant lui-même un générateur d'impulsions et des électrodes (105) reliées au générateur et appliquées au patient (102) au niveau d'un des points de passage du nerf moteur choisi tel le nerf cubital,

- d'un capteur (3) de la réponse musculaire à surveiller comprenant une jauge de contrainte et des moyens d'adaptation du capteur à l'organe (104) sur lequel on veut capter la réponse,

- d'un analyseur (109) du signal émis par le capteur (3), lesquels comprennent des moyens (18, 19) leur permettant d'émettre des stimulations différentes et d'analyser des réponses à ces stimulations différentes,

et de préférence,

- de moyens complémentaires (125, 126 et 127) d'information de l'analyseur (109) par le stimulateur (103) des instants où chacune des stimulations est émise,- d'un moyen (128) qui, en tenant compte de cette information et du retard entre l'émission d'une stimulation et la réception du signal de réaction correspondant, distingue dans le signal capté, avec une certaine tolérance, les périodes où se situent les éventuels signaux de réaction des autres périodes pendant lesquelles seuls sont captés des signaux parasites,

cet appareil qui comprend au moins un dispositif (1) permettant de maintenir en position adéquate le capteur (3) permettant de détecter le mouvement d'adduction du pouce (4) d'une des mains (5), lequel capteur comprend un piston coulissant par rapport à un corps au moins sensiblement cylindrique et enserré par la main du patient à la manière d'une poignée étant **CARACTERISE** en ce que le piston (35) du capteur (3) est associé au corps au moins sensiblement cylindrique de manière à coulisser sensiblement radialement à l'axe du corps pour, quelles que soient les dimensions de la main du patient, permettre l'actionnement du piston (35) du capteur non plus par la dernière mais la première phalange (10) du pouce (4) de la main (5) équipée du dispositif (1) et en ce que :

- le dit corps est associé à au moins une butée (7) disposée pour, en coopérant avec au moins l'un des organes que sont la main et l'avant bras du patient, établir une référence pour la position de la main (5) au long de l'axe longitudinal (8) de la poignée (6), et

- le dispositif comprend au moins un moyen (9) permettant d'ajuster sensiblement au long de l'axe (8) de la poignée (6), la position relative des éléments que sont la butée (7) et l'organe de détection (3).

2. Appareil selon la revendication 1 **caractérisé** en ce que l'axe longitudinal de la poignée (6) forme avec la butée (7) un angle (A) sensiblement supérieur à quatre vingt dix degrés.

3. Appareil selon la revendication 2 **caractérisé** en ce que angle (A) est sensiblement égal à cent cinq degrés.

4. Appareil selon la revendication 1 **caractérisé** en ce que, pour associer la butée (7) à l'extrémité (15) de la poignée opposée à celle (16) prés de laquelle est agencé le capteur, la dite poignée (6) est solidaire d'une platine (17) qui s'étend sensiblement parallèlement à l'axe de l'avant bras et cette platine, qui reçoit la butée (7) par sa face opposée (18) à celle d'appui (14), porte des moyens (19) pour sa fixation à la fraction de l'avant bras situé en vis à vis de la butée (7).

5. Appareil selon la revendication 1 **caractérisé** en ce que, pour l'adaptation des doigts de la main autour de la poignée, le dispositif comprend un coussin (21) de forme susceptible d'une part d'envelopper largement la main du patient en la contraignant élastiquement et ce au moins au niveau des doigts refermés sur la poignée mais d'autre part de dégager constamment la zone (22) du muscle adducteur (23) du pouce, lequel coussin est combiné à un élément support (211) relié à la poignée par un moyen de liaison (24) assurant le déplacement du coussin (21) au moins entre deux positions extrêmes (25, 26) dont une position (25) dans laquelle le coussin dégage totalement l'accés à la poignée pour sa préhension par le patient et une autre position (26) dans laquelle il enveloppe largement la main du dit patient comme précédemment défini pour maintenir ses doigts élastiquement refermés sur la poignée.

6. Appareil selon la revendication 5 **caractérisé** en ce que l'élément support (211) du coussin (21) consiste en un bouclier rigide qui enveloppe extérieurement le dit coussin (21) de manière à pouvoir induire un contact élastique optimal entre ce dernier et les doigts du patient refermés sur la poignée (6).

7. Appareil selon la revendication 1 comprenant un moyen (352) de liaison du pouce au piston, **caractérisé** en ce que le moyen de liaison (352) consiste en une sangle qui permet d'enserrer le pouce du patient et d'appliquer sur le piston une précharge de préférence réglable.

8. Appareil selon la revendication 1 **caractérisé** en ce qu'il comprend, en combinaison entre eux :

- un moyen (129) qui, pendant les périodes où seuls des signaux parasites peuvent être captés, mesure le niveau du signal capté et,

- un moyen (130) qui n'élimine du signal capté que la bande correspondant aux signaux de bruits mesurés, ce moyen (130) étant situé entre le capteur (3) et le moyen (128) de distinction des périodes pour former une boucle de réglage continu.

9. Appareil selon la revendication 1 dont le stimulateur (103) comprend un moyen (131) de réglage de l'intensité du signal émis et ce progressivement pour rechercher le niveau souhaité, **caractérisé** en ce que le stimulateur (103) comprend un moyen (132) de maintien du signal émis non pas à une tension constante mais à une intensité constante.

10. Appareil selon la revendication 1 comprenant un moyen de calibration (133) du signal capté permettant notamment d'adapter à la largeur de l'échelle de lecture un signal de référence propre à chaque cas **caractérisé** en ce que le moyen de calibration (133) consiste en un amplificateur à gain variable à réglage automatique.

_fig:1_

90

103

132
125
126
127
128
129
130
133
131   109
101

9   7   102
105
5   10
4
11   1   3   8   35
6

0283387

0283387

Fig. 2

Fig. 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | MEDICAL & BIOLOGICAL ENGINEERING & COMPUTING, vol. 23, no. 6, november 1985, pages 547-555, IFMBE, Londres, GB; H.S. BRADLOW et al.: "Microcomputer-based muscle relaxation monitor and controller for clinical use" * Abrégé; page 548, paragraphe: "2. Measurement system"; page 552, paragraphe: "4. Microcomputer system: software"; figures 1-5 * | 1,8,9 | A 61 B 5/10<br>A 61 B 9/00<br>A 61 N 1/36 |
| D,A | US-A-4 387 723 (J.L. ATLEE, III et al.) * Abrégé; colonne 11, lignes 9-52; revendications 1-5; figures 1-6 * | 1,7-9 | |
| A | US-A-4 541 417 (P.P. KRIKORIAN) * Colonne 7, ligne 27 - colonne 8, ligne 20; figure 6 * | 1,10 | |
| A | US-A-3 898 983 (J.O. ELAM) * Abrégé; figure 1 * | 1,4,5,7 | DOMAINES TECHNIQUES RECHERCHES (Int. Cl.4) |
| A | US-A-4 236 528 (A. STANEC et al.) * Abrégé; figures 1,2,11 * | 1,4,7 | A 61 B<br>A 61 N |
| A | ANESTHESIOLOGY, vol. 29, no. 5, septembre - octobre 1968, pages 1054 - 1055, Philadelphia, US; L.F. WALTS et al.: "A means of recording force of thumb adduction" * En entier * | 1 | |

Le présent rapport a été établi pour toutes les revendications

| Lieu de la recherche | Date d'achèvement de la recherche | Examinateur |
|---|---|---|
| LA HAYE | 15-06-1988 | HUNT,B.W. |